# EUROPÄISCHE PATENTANMELDUNG

(11) **EP 0 523 475 A1**
(43) Veröffentlichungstag der Anmeldung: **20.01.1993**
(21) Anmeldenummer: 92111299.1
(22) Anmeldetag: 03.07.1992
(51) Int. Cl.: C07C 41/16, C07C 43/178, C07C 47/575

(54) **Propargylether und Verfahren zu ihrer Herstellung**

(30) Priorität: 16.07.1991 DE 4123535
(60) Teilanmeldung: 94117013.6
(71) Anmelder: BAYER AG, 51368 Leverkusen (DE)
(72) Erfinder: Köhler, Burkhard, Dr., W-4150 Krefeld 11 (DE); Dujardin, Ralf, Dr., W-4156 Willich 2 (DE); Ebert, Wolfgang, Dr., W-4150 Krefeld (DE)

(57) **Zusammenfassung**

Die Erfindung betrifft ein Verfahren zur Herstellung von Progargylethern mit Eisenkatalyse und Propargylether der allgemeinen Formel I 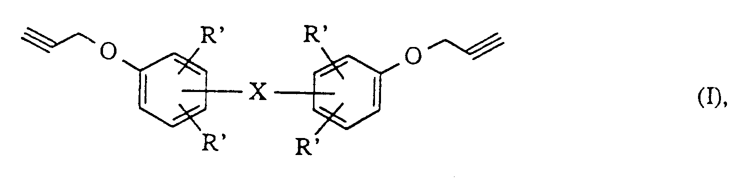
in der
R' für Wasserstoff oder Methyl und X für eine cycloaliphatische Gruppe steht.

## Beschreibung

Die Erfindung betrifft ein Verfahren zur Herstellung neuer Propargylether mit Eisenkatalyse und neue Propargylether.

Propargylether sind für die Herstellung von hydrophoben Duromeren von Interesse, die die Vorteile der Epoxyharze mit dem Vorteil geringer Wasseraufnahme verbinden. In High Performance Polymers 2(1) (1990), 67-77 wird diese Duromerklasse und die herkömmlichen Verfahren zur Herstellung der als Bausteine verwendeten Propargylether beschrieben. Einige der Verfahren führen zur Bildung der unerwünschten Claisen-Umlagerungsprodukte, andere Verfahren benötigen lange Reaktionszeiten.

Daher sind Verfahren von Interesse, die eine kurze Reaktionsdauer bei schonenden Bedingungen ermöglichen.

Ferner sind neue Propargylether mit hohem Cycloaliphatenanteil von Interesse, die die Herstellung von potentiell noch hydrophoberen Duromeren ermöglichen.

Überrachenderweise wurde nun gefunden, daß die Umsetzung von Phenolen, Bisphenolen oder Polyphenolen mit Propargylhalogeniden durch Zusatz von Eisen oder Eisenverbindungen beschleunigt wird.

Gegenstand der Erfindung ist daher ein Verfahren zur Herstellung von aromatischen Propargylethern, in dem phenolische Verbindungen, vorzugsweise Mono-, Di-, oder Polyphenole mit
bezogen auf 1 Mol phenolische OH-Gruppen 1-2 Mole Propargylhalogenid in Gegenwart von
bezogen auf 1 Mol phenolische OH-Gruppen 1-2 Mole Base
in Lösungsmitteln, vorzugsweise in aliphatischen C1-C4- Alkoholen, wie z.B. Isopropanol oder in Ketonen, wie z.B. Aceton, Methylethylketon, Cylohexanon, oder in Nitrilen, wie z.B. Acetonitril, oder in Amiden, wie z.B. Dimethylformamid oder Dimethylacetamid, oder in Lactamen, wie z.B. N- Methylpyrrolidinon, N-Methylcaprolactam, oder in Harnstoffen, wie z.B. Tetramethylharnstoff, N,N'-Dimethylpropylenharnstoff, N,N'-Dimethylimidazoldidinon oder in Dimethylsulfoxid,
umgesetzt werden, dadurch gekennzeichnet, daß der Reaktionslösung, bezogen auf 1 Mol phenolische OH-Gruppen, 0,0001-0,01 Mol Eisen in elementarer Form oder in Form von Eisenverbindungen, vorzugsweise von EisenII- oder EisenIII-salzen, wie z.B. Oxiden, Sulfiden, Halogeniden, Nitraten, Sulfaten, Formiaten, Oxalaten, Tartraten, Acetaten, aliphatischen C₃-C₂₂- Carboxylaten, aromatischen C₆-C₁₄- Carboxylaten, besonders bevorzugt in Form einer im Lösungsmittel der Reaktion löslichen Eisenverbindung, zugesetzt werden.

Ein weiterer Gegenstand der Erfindung sind neue Proparglyether der allgemeinen Formel I 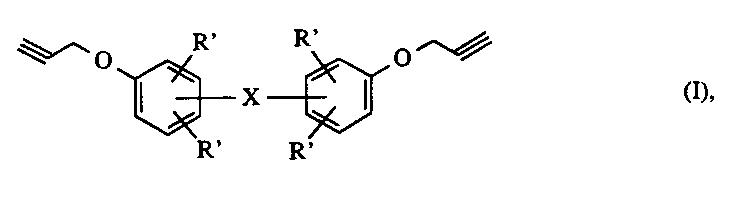
in der
- R': für Wasserstoff oder Methyl und
- X: für die Reste

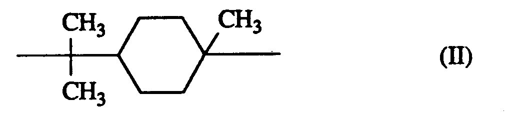

oder 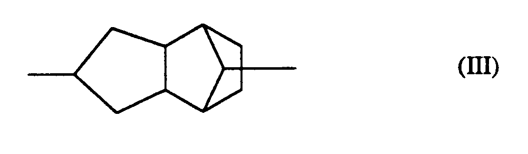
oder 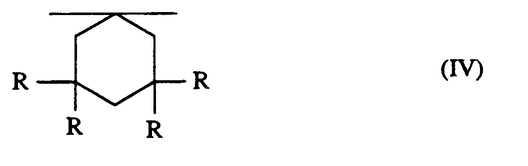
steht,
in dem
- R: für Wasserstoff oder C₁-C₂₂-Alkyl steht.

Bevorzugter Rest X ist der Rest der Formel (IV). Die neuen Progarglyether werden bevorzugt erhalten nach dem erfindungsgemäßen Herstellungsverfahren.

Beispiele für erfindungsgemäß umsetzbare Monophenole sind Phenol, Kresole, Xylenole, 2- oder 4-Phenylphenol, Octylphenole, Nonylphenole, Dodecylphenole, 2- Hydroxybenzaldehyd, 4-Hydroxybenzaldehyd, 2-, 3- oder 4-Hydroxybenzoesäure oder deren Ester oder Amide, 4-Hydroxyzimtsäure oder deren Ester oder Amide.

Beispiel für erfindungsgemäß umsetzbare Diphenole sind Bishydroxyphenylmethane (alle möglichen Isomeren), 2,2-Bis(4-hydroxyphenyl)propan, 2,2-Bis(4-hydroxyphenyl)hexafluorpropan, Hydrochinon, Resorcin, Brenzkatechin, 4,4'-Dihydroxybiphenyl, 2,2'-Dihydroxybiphenyl, Dihydroxyterphenyl, Dihydroxyquaterphenyl, 4,4'- oder 3,3'-Dihydroxdiphenylether, 4,4'-Dihydroxydiphenylsulfon, 4,4'-Dihydroxydiphenylsulfid, 3,4- oder 2,4-Dihydroxybenzaldehyd, 2,4- oder 2,4'- oder 4,4'- Dihydroxybenzophenon, 2,4- oder 3,4-Dihydroacetophenon, 4,4'-Dihydroxystilben, 1,2-Bis(4-hydroxyphenol)ethan, 1,2-Bis(4-hydroxyphenyl)propan.

Beispiele für erfindungsgemäß umsetzbare Diphenole, wobei die Umsetzung zu den erfindungsgemäß Proparglyethern mit hohem Anteil an cycloaliphatischen Gruppen führt, sind Bis(4-hydroxyphenyl)cyclohexan, 1,1-Bis(4-hydroxyphenyl)-3,3,5-trimethylcyclohexan, Kondensationsprodukte aus Phenol und Dicyclopentadien, Kondensationsprodukte aus Phenol und Terpenen, wie z.B. Limonen, besonders bevorzugt 1,1-Bis(4- hydroxyphenyl)-3,3,5-trimethylcyclohexan.

Beispiele für erfindungsgemäß umsetzbare Polyphenole sind Kondensationsprodukte aus Formaldehyd und Phenolen, wie z.B. Novolake.

Beispiele für erfindungsgemäß verwendete Basen sind Alkalialkoholate, Alkalicarbonate, Alkalihydroxide, tertiäre Amine oder basische Ionenaustauscher.

Beispiele für erfindungsgemäß verwendete Lösungsmitte sind Methanol, Ethanol, n-Propylalkohol, Isopropylalkohol, Butanole, Aceton, Cyclohexanon, Acetonitril.

Das erfindungsgemäß Verfahren kann innerhalb von 0,1 bis 10 Stunden, vorzugsweise 0,5 bis 5 Stunden, bei Temperaturen von 0°C bis 100°C, vorzugsweise von 20°C-80°C, und bei einem Druck von 0,5 bis 10 bar, vorzugsweise bei Normaldruck, durchgeführt werden.

Es zeichnet sich durch eine hohe Reaktionsgeschwindigkeit aus. Ferner treten unerwünschte Umlagerungen nur in geringem Ausmaß auf.

Die erfindungsgemäßen Proparglyether mit hohem Anteil an cycloaliphatischen Gruppen zeichnen sich durch eine gute Löslichkeit sowohl in polaren Lösungsmitteln, wie z.B. Isopropanol, als auch in unpolaren Lösungsmitteln, wie z.B. Petrolether, aus. Eine gute Löslichkeit erleichtert die Verarbeitung zu Glas-oder Kohlenstoffaser-verstärkten Duromerbauteilen, wobei die Verstärkungsfaser vorher mit einer Monomerlösung getränkt werden muß.

### Beispiele

### Vergleichsbeispiel 1

### Herstellung von 4-Proparglyoxybenzaldehyd

Man mischt 122 g 4-Hydroxybenzaldehyd, 1000 ml Aceton, 138 g Kaliumcarbonat und 150 g Propargylbromid. Man erhitzt 4 Stunden auf 60°C, filtriert ausgefallene Bestandteile ab, engt ein, nimmt in Methylenchlorid auf, schüttelt mit 10 proz. NaOH aus und engt die organische Phase ein. Man erhält 20 bis 35 g 4-Proparglyoxybenzaldehyd.

### Beispiel 1 (erfindungsgemäßes Verfahren)

### Herstellung von 4-Proparglyoxybenzaldehyd

Man verfährt wie in Vergleichsbeispiel 1 beschrieben, setzt aber der Mischung 0,05 g Eisen(III)chlorid zu. Man erhält 109 g 4-Proparglyoxybenzaldehyd in NMR-reiner Form.

### Beispiel 2 (erfindungsgemäßes Verfahren)

### Herstellung von 2,2-Bis(4-Proparglyoxyphenyl)propan

Man mischt 228 g Bisphenol-A, 300 ml Isopropanol, 360 g einer 30 proz. Lösung von Natriummethanolat in Methanol, 0.05 g Eisen(III)chlorid und 200 g Proparglychlorid. Es kommt zu einer exothermen Reaktion, nach deren Dauer von ca. 15 min die Hauptmenge des Produktes entstanden ist (DC-Kontrolle). Man läßt 4 Stunden nachreagieren, filtriert ab und engt ein. Man erhält 275 g des Produktes (durch NMR-Spektroskopie charakterisiert).

### Beispiel 3 (Proparglyether mit hohem Cycloaliphatenanteil)

### Herstellung von 1,1-Bis(4-proparglyoxyphenyl)-3,3,5- trimethylcyclohexan

Man verfährt wie in Beispiel 2 beschrieben, wobei das Bisphenol-A durch 319 g 1,1-Bis(4-hydroxyphenyl)-3-3-5- trimethylcyclohexan ersetzt wird. Man erhält 326 g Produkt (durch NMR-Spektroskopie charakterisiert).

## Patentansprüche

1. Verfahren zur Herstellung von aromatischen Proparglyethern durch Umsetzung von phenolischen Verbindungen mit 1 bis 2 Molen Propargylchlorid, bezogen auf 1 Mol phenolische OH-Gruppen, in Gegenwart von 1 bis 2 Molen Base, bezogen auf 1 Mol phenolische OH-Gruppen in organischen Lösungsmitteln, dadurch gekennzeichnet, daß die Umsetzung in Gegenwart von Eisenkatalysator durchgeführt wird.

2. Verfahren nach Anspruch 1, dadurch gekennzeichnet, daß als phenolische Verbindungen Mono-, Di- oder Polyphenole eingesetzt werden.

3. Verfahren nach Anspruch 1, dadurch gekennzeichnet, daß als organische Lösungsmittel C₁-C₄- Alkohole, Ketone, Nitrile, Amide Lactame oder Harnstoffe eingesetzt werden.

4. Verfahren nach Anspruch 1, dadurch gekennzeichnet, daß als Eisenkatalysator von 0,0001 bis 0,1 Mol Eisen, bezogen auf 1 Mol phenolische OH-Gruppen in elementarer Form oder in Form von Eisenverbindungen eingesetzt wird.

5. Verfahren nach Anspruch 4, dadurch gekennzeichnet, daß als Eisenkatalysator Eisen II- oder EisenIII-Salze eingesetzt werden.

6. Verfahren nach Anspruch 1, dadurch gekennzeichnet, daß die Umsetzung bei 0 bis 100°C durchgeführt wird.

7. Proparglyether der allgemeinen Formel I 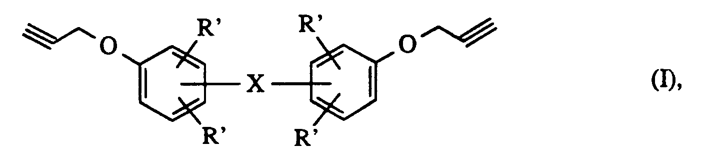 in der
R' für Wasserstoff oder Methyl und
X für die Reste 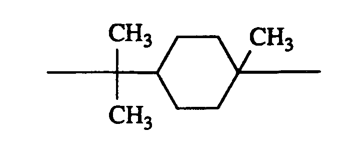
oder 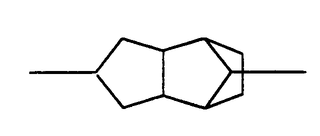 oder 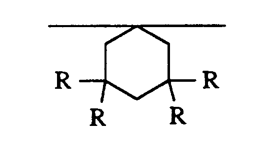 steht,
in dem
R für Wasserstoff oder C₁-C₂₂-Alkyl steht.

8. Proparglyether nach Anspruch 7 der allgemeinen Formel I 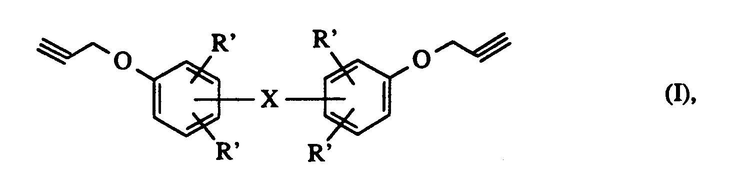 in der
X für den Rest 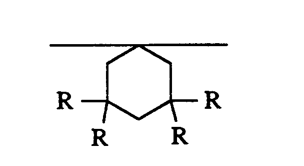
steht,

9. Proparglyether nach Anspruch 7, hergestellt nach dem Verfahren gemäß Anspruch 1.
